# EUROPEAN PATENT APPLICATION

(11) **EP 1 211 260 A1**
(43) Date of publication of application: **05.06.2002**
(21) Application number: 00204268.7
(22) Date of filing: 30.11.2000
(51) Int. Cl.: C07K 14/35, A61K 39/04, C12N 15/31

(54) **Peptides from Ag85 of mycobacterium and uses thereof**

(71) Applicant: Academisch Ziekenhuis Leiden, 2333 ZA Leiden (NL)
(72) Inventor: Ottenhof, Thomas Henricus Maria, 2334 CJ Leiden (NL); Geluk, Annemieke, 2481 XA Woubrugge (NL)
(74) Representative: Prins, Adrianus Willem

(57) **Abstract**

The present invention relates to the field of immunology. In particularly the invention provides a peptide derived from an Ag85 protein of mycobacterium comprising 8-11 amino acid residues, said peptide capable of inducing proliferation of MHC class I-restricted CD8⁺ T cells *in vivo*. One example for use of the present invention is a novel vaccine composition against mycobacterium.

## Description

The invention relates to the field of immunology. In particularly the invention relates to a peptide derived from mycobacterium and uses thereof.

Tuberculosis is a re-emerging disease that represents a major public health problem in many countries, especially in the developing world (1). *Mycobacterium tuberculosis* is primarily transmitted via the respiratory route and causes disease in 5-10% of infected individuals, causing approximately 3 million deaths annually.

Although the mechanisms of protection against tuberculosis are not yet completely understood, effective cell-mediated immunity is essential to control infection with *M. tuberculosis.* Many studies have indicated a prominently protective role for CD4⁺ T cells (3), and several HLA-class II-restricted epitopes have been identified on proteins of *M. tuberculosis* (4,5). Since it was reported that β₂m-deficient mice, which lack CD8⁺ T cells, show increased susceptibility to experimental tuberculosis (6), the role of CD8⁺ T cells has drawn increasing attention. For CD1-restricted, CD8⁺ T cells several non-protein ligands have recently been identified. These CD8⁺ T cells were capable of lysing *M. tuberculosis*-infected target cells and concomitantly kill intracellular pathogens via a granule-exocytosis pathway (7), yet their precise contribution in intracellular infections remains unknown. The recent application of DNA vaccines to tuberculosis has provided evidence for MHC class I-restricted, CD8⁺ T cell-mediated protection in mouse models of tuberculosis (8,9). In humans the existence of mycobacterium-reactive MHC class I-restricted CD8⁺ T cells has been demonstrated (10, 11), but very little is known about which antigens are recognized by such T cells. Only two mycobacterial epitopes that are recognized by HLA class I-restricted CD8⁺ T cells have been reported: an HLA-B52-restricted epitope of *M. tuberculosis* ESAT-6 (12) and an HLA-A*0201-restricted peptide of the 19 kDa lipoprotein (13).

The currently available means for controlling tuberculosis are inadequate: the highly variable protection induced by the commonly used vaccine *M.bovis* BCG (2), together with the HIV pandemic and the increasing multidrug-resistance in *M. tuberculosis* strains have highlighted the need for new effective vaccines.

The present invention as disclosed herein provides novel components for, for example, the composition of a vaccine against mycobacterium.

In a first embodiment the invention provides a peptide derived from an Ag85 protein of mycobacterium comprising 8-11 amino acid residues, said peptide capable of inducing proliferation of MHC class I-restricted CD8⁺ T cells *in vivo.* Secreted, extracellular antigens are likely to be important in the induction of protective immunity, especially during the early phase of infection. Up to thirty percent of *M. tuberculosis* culture filtrate proteins is composed of antigen 85 (Ag85), a family of three highly homologous, 30-32kD proteins: 85A, 85B and 85C. Each of these antigens is associated with mycolyltransferase activity *in vitro,* suggesting their essential involvement in the synthesis of the characteristic cell wall of mycobacteria. Ag85 induces strong T cell proliferation and IFN-γ secretion in most healthy individuals exposed to *M. tuberculosis,* in BCG-vaccinated mice and humans, whereas Ab against Ag85 are more prevalent in active tuberculosis patients with decreased cellular immune response. Furthermore, vaccination with plasmid DNA encoding the 85A or 85B component generated strong Th1-type, CD8⁺-mediated immune responses and induced protection against *M. tuberculosis* challenge in mice and guinea pigs. While it is well known that CD4⁺ T cells are crucial in the protection against infectious disease caused by *M. tuberculosis,* the contribution of CD8⁺ T cells has been less well established in this respect. A protective role for CD8⁺ T cells, has been suggested earlier in experimental tuberculosis in mice, since depletion of CD8⁺ T cells or adoptive transfer thereof was accompanied by a reduction of the number of live *M. tuberculosis* bacteria detected in infected organs. In addition, β2m^{-/-} mice, which lack CD8⁺ T cells, were shown to be highly susceptible to tuberculosis. Therefor CD8⁺ T cells are thought to play an important role in protective immunity to tuberculosis. Although several non-protein ligands have been identified for CD1-restricted CD8⁺ CTLs, epitopes for classical MHC class I-restricted CD8⁺ T cells, that likely represent a majority among CD8⁺ T cells, have remained ill-defined and it was not known until the present invention that a peptide derived from an Ag85 protein of mycobacterium comprising 8-11 amino acid residues is capable of inducing proliferation of MHC class I-restricted CD8⁺ T cells *in vivo.* It is clear to a person skilled in the art that a peptide according to the invention can be produced by different means, for example, synthetically or produced by a known overexpression system. It is clear to a person skilled in the art that a modification to a peptide according to the invention, for example an amino acid substitution, is also included as long as the modified peptide is still capable (possibly in different amounts) of inducing proliferation of MHC class I-restricted CD8+ T cells *in vivo.* Therefor a functional derivative of a peptide according to the invention is included by this invention. It is important to emphasise that the proliferation of MHC class I-restricted CD8⁺ T cells by a peptide according to the invention occurs under *in vivo* circumstances, because experiments as disclosed herein have indicated that DNA immunization could also induce cells directed against peptides that are only processed following DNA plasmid immunization but not following whole antigen pulsing or BCG infection.

Furthermore a peptide according to the invention could contain flanking amino acids representing antigen processing sites and therefor the invention also provides a peptide comprising a peptide, which is derived from an Ag85 protein of mycobacterium comprising 8-11 amino acid residues, said peptide capable of inducing proliferation of MHC class I-restricted CD8⁺ T cells *in vivo,* flanked by amino acids representing antigen processing sites. In yet another embodiment the invention provides a polypeptide comprising at least 2 peptides comprising such flanking amino acid sequences representing antigen processing sites. Examples of such a polypeptide and procedures how to arrive at such a polypeptide are known in the art. One example of a polypeptide is a string-bead polypeptide wherein peptides according to the invention are separated by amino acids. The amino acids separating the (different) peptides can be subjected to cleavage in a cell and thereby providing multiple (different) peptides.

In a preferred embodiment the invention provides a peptide or polypeptide according to the invention wherein said peptide is derived from an Ag85B protein. The Ag85 protein of *M.tuberculosis* is a family of three highly homologous, 30-32 kDa proteins: 85A, 85B and 85C. Each of these antigens is associated with mycolyltransferase activity *in vitro,* suggesting their essential involvement in the synthesis of the characteristic cell wall of mycobacteria. Peptides according to the invention are strongly conserved between *M.tuberculosis* Ag85B and Ag85A, and the corresponding Ag85A peptides had comparable binding affinities for the tested MHC class I molecules. Therefor Ag85A and Ag85B protein can both be used to provide a peptide or polypeptide according to the invention.

In a more preferred embodiment the invention provides a peptide or polypeptide according to the invention wherein said Ag85 protein of mycobacterium is selected from the group consisting of *M. tuberculosis, M. leprae, M. bovis, M. ulcerans* and *M. avium.* Peptides according to the invention derived from Ag85 may represent a promising antigenic subunit for anti-mycobacterial vaccination since it is widely expressed by mycobacteria, including the pathogens *M. tuberculosis, M. leprae, M*. *bovis, M. ulcerans* and *M. avium,* whereas no human homologue exists for Ag85, thus avoiding the risk of auto-reactivity *in vivo.*

In a even more preferred embodiment the invention provides a peptide or polypeptide according to the invention wherein said MHC class I-restricted CD8⁺ T cells are HLA-A*0201- restricted CD8⁺ T cells. Since the antigen specificity of the human T cell response is known to be strongly controlled by HLA polymorphism, the immunogenic potential of candidate vaccines needs to be defined in the context of major HLA polymorphism. Proper tools to examine the influence of HLA polymorphism *in vivo* have become available only recently by the generation of HLA tg mice. Disclosed herein is the use of DNA vaccination of HLA-A2/K^{b} tg mice to examine the *in vivo* induction and specificity of CD8⁺ T cell responses against *M. tuberculosis* in the context of a major human MHC class I allele. Peptide-specific CD8⁺, HLA-A*0201-restricted cytotoxic T cells in HLA-A*0201⁺ mice and humans are disclosed in this description. CD8⁺ T cells recognizing Ag85 p143-152 and p199-207 were detected in HLA-A2/K^{b} tg mice and HLA-A*0201⁺, BCG-responsive individuals, but not in HLA-A*0201⁻ donors. Importantly, these Ag85B-reactive CD8⁺ T cells not only lysed peptide- or Ag85B-pulsed target cells but also BCG-infected HLA-A*0201⁺ human macrophages. This shows that Ag85B p143-152 and p199-207 are naturally processed from mycobacteria after infection

In yet another embodiment the invention provides a nucleic acid encoding a peptide or a polypeptide according to the invention. Furthermore the invention provides a vector comprising a nucleic acid according to the invention. A nucleic acid or a vector according to the invention is, for example, used for the production of a DNA-based vaccine. In another embodiment the invention provides a host cell comprising a nucleic acid or vector according to the invention.

In a preferred embodiment the invention provides the use of a peptide or polypeptide, a nucleic acid, a vector according to the invention for the preparation of a vaccine against mycobacterium. Because Ag85 from which a peptide or polypeptide according to the invention is derived, is widely expressed by mycobacterium, including *M. tuberculosis, M. leprae, M. bovis, M. ulcerans* and *M. avium,* the finding as disclosed herein is used to design a vaccine against different mycobacteria. Methods for the preparation of a suitable vaccine composition are well known by a person skilled in the art. A vaccine is for example peptide or polypeptide based. In a peptide or polypeptide (for example a string-bead polypeptide) vaccine the immunogen is a single peptide or a small assembly of peptides. Since most immune responses against peptide or polypeptide vaccines are T-cell dependent and because T cell recognition is MHC-restricted and given the wide polymorphism of the different MHC molecules, distinct epitopes may be recognised by different individuals in a population. In a polypeptide vaccine the peptides may be identical or different and by introducing different peptides via a polypepide it is possible to prepare a vaccine composition which can be recognized by different individuals in a population and therefor the effectiveness of such a polypeptide-based vaccine will be higher. In another example a vaccine composition can comprise MHC-molecules loaded with a peptide or polypeptide according to the invention or a vaccine composition can comprise antigen presenting cells loaded with a peptide or a polypeptide according to the invention. Examples of antigen presenting cells are macrophages or dendritic cells. A DNA-based vaccine composition is in principle one of the most simple and yet versatile methods of inducing an immune response and therefor a vaccine based on a nucleic acid or vector encoding a peptide or polypeptide according to the invention is also included. When needed to obtain a good immunogenic response a vaccine composition can also comprise an adjuvant. A person skilled in the art knows when and how to select a proper adjuvant to obtain a vaccine compositions which is capable of eliciting an effective immunogenic response.

In yet another embodiment the invention provides a method to detect and/or enumerate CD8⁺ T cells against mycobacterium comprising tetrameric complexes of MHC class I and a peptide or polypeptide according to the invention. Experiments as disclosed herein have shown that tetrameric complexes of MHC class I molecules and a peptide or a polypeptide according to the invention specifically show a staining signal in case of BCG responsiveness in the context of a MHC class I molecule. Methods to arrive at such a tetrameric complex are known in the art. These data provide a novel tool for detection and enumeration of *M.tuberculosis-specific* CD8⁺ T cells.

The invention will be explained in more detail in the following detailed description which is not limiting the invention.

### EXPERIMENTAL PART

### Peptide selection and synthesis.

Candidate HLA-A*0201 binding peptides in Ag85B were selected using *MOTIFS* software (15). Positive scores were given for each potential anchor residue found in the peptide, and negative scores were given to inhibitory residues. The overall peptide score was the sum of the scores for individual anchor and inhibitor residues. Scores ranged from -9 until 65. All 8-, 9-, 10-and 11-mer peptides scoring ≥ 45 were synthesized as described previously (4).

### HLA-A*0201-peptide binding assay

Recombinant HLA-A*201 was overexpresssed in *E. coli,* purified as described (16) and dissolved in 8M urea. HLA-A2*0201 was titered in the presence of 100 fmol standard peptide to determine the HLA concentration necessary to bind 20-50% of the total fluorescent signal. All subsequent inhibition assays were performed at this concentration. HLA-A2*201 was incubated in 96-well serocluster plates (Costar) at 20°C for 48 h with 0.5 µl β₂m (15 pmol) and 1 µl (100 fmol) fluorescent labeled peptide in 92.5 µl assay buffer (100 mM Na-phosphate, 75 mM NaCl, 1 mM CHAPS, pH 7), 2 µl protease inhibitor mixture (1 µM chymostatin, 5 µM leupeptin, 10 µM pepstatin A, 1 mM EDTA, 200 µM pefabloc) and 2 µl of test peptide. As a standard peptide HBV core 47-56 (52->C) was used. The HLA-peptide complexes were separated from free peptide by gel filtration on a Synchropak GPC 100 column (250mm x 4.6mm; Synchrom, Inc., Lafayette, Indiana) using assay buffer containing 5% CH₃CN. Fluorescent emission was measured at 528 nm on a Jasco FP-920 fluorescence detector (B&L Systems, Maarssen, The Netherlands). The percentage of labelled peptide bound was calculated as the amount of fluorescence bound to MHC divided by total fluorescence. The concentration of peptide inhibitor yielding 50% inhibition was deduced from the dose-response curve.

### Ag85B protein.

Ag85B protein was obtained by cloning the gene coding for Ag85B in pET19b vector (Novagen, Madison, WI) using PCR. The protein was expressed as a fusion protein, containing 10 histidine residues plus a 13 amino acid containing linker sequence attached to its N-terminus. For over-expression *E.coli* B strain BL21 (DE3) (17) was used in which the T7 RNA polymerase gene is under control of the *lac*UV5 promoter. Expression in *E.coli* was induced at an OD₆₀₀ of 0.6 by addition of 1mM isopropyl-D-thiogalacto-pyranoside. The cells were harvested after 5h culture at 37°C and centrifuged at 5000xg for 15 min. Ag85B protein was purified by Ni-chelate affinity chromatography (Qiagen, Chatsworth, CA) (18).

### Plasmid construction.

Plasmid DNA encoding Ag85B was prepared as described previously (9). Briefly, the gene from plasmid pAg85B (19) of *M. tuberculosis* was amplified without its mycobacterial signal sequence by PCR with *Bgl*II site containing primers. Amplified DNA was digested with *Bgl*II isolated on a 1% agarose gel, and extracted on Prep A Gene (Bio-Rad). Fragments were ligated to the *Bgl*II-digested and dephosphorylated V1J-ns-tPA vector, transformed into competent *E. coli* DH5 (Biological Research Labs, Breda, The Netherlands) cells and plated on LB (Luria-Bertani) agar medium containing kanamycin (50 µg/ml). Recombinant plasmid DNA was amplified in *E.coli* DH5 and purified on two CsCl₂-ethidium bromide gradients, extracted with 1-butanol and phenolchloroform, and precipitated with ethanol. In this plasmid, the Ag85B gene is expressed under the CMV promoter of IE1 form preceded by a tPA leader sequence, and followed by a polyadenylation site of the bovine growth hormone.

### Mice.

HLA-A*0201/K^{b} (HLA-A2/K^{b}) transgenic mice (14) were kindly provided by Dr. L. Sherman (Scripps Laboratories, San Diego, CA) and bred under specific pathogen free conditions at TNO-PG, Leiden, The Netherlands. Besides the H2-K^{b} and H2-D^{b} molecules, these mice express a chimeric HLA-A*0201/K^{b} gene encoding the murine H-2K^{b} a3 domain and the HLA-A*0201 a 1 and a2 domains. This allows the murine CD8 molecule on the murine CD8⁺ T cells to interact with the syngeneic a3 domain of the hybrid MHC class I molecule. Surface expression of the HLA-A*0201/K^{b} molecule was confirmed by FACS-analysis.

### Immunizations.

Mice were anesthesized by i.p. injection of ketamine/xylazine (100 mg/kg and 10 mg/kg, respectively) and injected i.m. 3 times (at 3-wk intervals) in both quadriceps (2x 50 µl) with Ag85B plasmid (1 mg/ml) or control DNA (empty vector) in PBS. Splenocytes were harvested three weeks after the last DNA injection. For peptide immunizations, equal volumes of peptide in PBS and IFA (DIFCO, Detroit, MI) were administered subcutaneously in the base of the tails, and cells were harvested seven days postinjection.

### Cytotoxicity assays.

The human EBV-BLCL JY (HLA-A*0201, -B7, -Cw7) was incubated at 37° C for 1 h with 0.1 mCi Na⁵¹Cr (Amersham, UK), washed and plated with effector cells in triplicates in 96-well round-bottomed plates (2500c /well) together with medium, peptide (2 µg) or Triton 5%. After 6 h supernatants were harvested and % specific lysis was calculated as: (release - spontaneous release) / (maximum release - spontaneous release) x 100%.

### CD4-depletion.

After peptide immunizations, splenocytes were incubated with antimouse CD4 mAb (GK1.5) for 30 min at 4°C, washed and added to a 10-fold excess of magnetic beads coupled to goat-anti-mouse IgG (Dynal, Norway). After 30 min at 4°C beads were removed from the cell suspension and the cells were checked for expression of CD4 and CD8 by FACs analysis. Efficiency of depletion was > 95%.

### Proliferation tests.

Splenocytes or lymphocytes (10⁶c/well) in RPMI 1640 (Life Technologies, Rockville, MD)-10% heat-inactivated FCS were added to 96-well flat-bottomed plates and in triplicates stimulated with antigen. For Ag85B presentation APC were treated by hypertonic shock (29) to provide excess to the class I pathway. After 24 h, 1 µCi [³H]thymidine was added. After 18 h, radioactivity incorporated into the DNA was determined by liquid scintillation counting. For blocking experiments the following antibodies were used: BB7.2 (anti-HLA-A), FK18 (anti-CD8), B8.11.2. (anti-HLA-DR).

### Determination of anti-Ag85 antibodies.

Ab-levels of serum from immunized mice were determined by ELISA as described previously (9). Serum titer was converted to Ab concentration by comparison with standard Ag85 specific mAb (17-4). Mean Ab concentration was calculated from three points of the linear part of the titration curve.

### Generation of Ag85B-specific, human CTL.

Human PBMCs, from healthy HLA-A*0201⁺ individuals, were depleted of T lymphocytes by using SRBCs followed by adherence in 6-well plates (Costar Corp., Cambridge, MA) at 37 °C in IMDM-10% FCS (Life Technologies) (10⁷c/well). After 2 h, GMCSF (800 u/ml) and IL-4 (500 u/ml) (Genzyme) were added, followed after 4 days by the addition of SACS culture medium (Staphylococcus Aureus culture supernatant) (21) to accomplish maturation of dendritic cells (DCs). Mature DCs were then pulsed with Ag85B peptides for 6 h. Autologous T lymphocytes were enriched for CD8⁺ T cells using anti-CD8-labeled MACS Magneticbeads (Miltenyi Biotec GmbH, Bergisch Gladbach, Germany). DCs were pulsed with peptide (10 µg/ml), washed and added to CD8-enriched T cells in IMDM-10% human serum (HS), containing IL-12 (100 pg/ml) and IL-7 (10 ng/ml) (Genzyme). After 7 days rIL-2 (25 u/ml) (Cetus, Amsterdam, The Netherlands) was added. T cells were checked weekly for CD8 expression by FACs analysis and restimulated (for 4-6 rounds) using autologous, peptide-pulsed PBMCs.

### Generation of BCG-activated STCL.

PBMCs (3x10⁶c/well) were incubated in 24-well for 2 h in antibiotic-free culture medium. After removal of non-adherent cells, BCG was added for 16 h at a multiplicity of infection (MOI) of 2:1, mycobacteria to macrophages. Non-adherent cells were enriched for CD8⁺ T cells (MACS Magneticbeads) and activated by addition to BCG-infected adherent cells.

### Cytokine assays.

TNF-α production was detected by addition of 24 h-supernatants from 2x10⁴ target cells BLM (HLA-A*0201⁺ melanoma cell line) and 2500 effector cells to WEHI cells as described (22). IFN-γ levels were determined after 72 h in supernatants from 3x10⁴ target cells (JY) and 2x10⁴ effector T cells. Detection capture mAb and polyclonal detection Ab for IFN-γ were obtained from P. van der Meide (UcyTech, Utrecht, NL) (sensitivity 3pg/ml). For Ag85B presentation APC were first treated by hypertonic shock (20).

### BCG infection of monocytes.

PBMCs (3x10⁵c/well) were cultured in 96-well flat bottomed plates for 2 h in antibiotic-free culture medium. After removal of non-adherent cells, BCG (MOI ≅ 2:1; estimating 3x10⁴ mφ/well) was added for 16 h. Adherent cells were washed with medium (37°C) before addition of Ag85B-specific CD8⁺ T cells (8x10³c/well). An aliquot of infected monocytes was analyzed to verify infection by acid-fast staining.

### Tetramers.

MHC/peptide tetramers were produced as described (23), but to abrogate CD8 binding, a mutated HLA-A*0201 heavy chain (denoted A2m) was used, containing the mutation A₂₄₅→V. Briefly, rA2m heavy chain and rβ2m were produced as inclusion bodies in *E.coli* XA90F'LacQ1, washed extensively, dissolved in 8M urea and refolded as HLA monomeric proteins in the presence of 12.5 mmol of Ag85B p199-207 in 100 mM Tris, 0.4M Arginine, 2mM EDTA, pH8.0, 0.5mM GSSG, 5mM GSH and protease inhibitors. Monomers were concentrated, dialyzed (Tris 10mM, pH 8.0) and biotinylated with 6µg/ml of BirA enzyme for 4 h at 30 °C. Biotinylated complexes are dialyzed and purified by ion exchange chromatography (monoQ, Pharmacia France, St. Quentin en Yvelines, France) to remove free biotin. Tetramerization was achieved by addition of PE-conjugated Streptavidin (Immunotech, Marseille, France) in a ratio of 4:1, and controlled by gel filtration on a Superdex 200 (Pharmacia).

### Tetramer staining.

CD8-enriched STCLs (10⁵/well) were incubated in V-bottomed 96-well plates, washed twice (PBS, 1% FCS) and stained for 1 h at 4°C with PE-labelled tetramers (1 µg) and PerCP-labeled anti-human CD8 (Becton Dickinson, CA), washed and analyzed by flow cytometry on a FACScalibur analyzer (Becton Dickinson).

### RESULTS

### 1. Identification of high affinity HLA-A*0201 binding peptides in the sequence of M. tuberculosis Ag85.

Immunodominant T cell epitopes often display high binding affinity for MHC molecules (24). Since HLA-A*0201 is one of the most prominent HLA class I alleles, we selected thirty candidate peptide epitopes (8-, 9-, 10-, and 11-mers) from the *M. tuberculosis* Ag85B sequence based on the presence of a described HLA-A*0201 peptide binding motif (15). Peptides were synthesized and tested for binding to HLA*0201 molecules. Six of the predicted peptides bound to HLA-A*0201 with high affinity (IC₅₀ < 1µM), 10 with intermediate affinity (IC₅₀: 1-10 µM) and 14 peptides bound weakly or not at all (IC₅₀ >10 µM) (Table I). The 16 highest affinity HLA-A*0201 binding peptides were used in the subsequent study.

**TABLE I**

| *Binding to HLA-A*0201 of M. tuberculosis Ag85B peptides* | | | | |
|---|---|---|---|---|
| Position | AA Sequence* | HLA-A*0201 Binding Motif Score | HLA-A2 Binding Affinity(IC₅₀; µM)^{¶} | Recognition by murine CTL^{§} |
| 143-152 | FIYAGSLSAL | 56 | 0.01 | + |
| 65-72 | G**L**SIVMP**V** | 60 | 0.3 | - |
| 143-151 | F**I**YAGSLS**A** | 49 | 0.4 | - |
| 126-135 | S**M**AGSSAMI**L** | 50 | 0.5 | + |
| 199-209 | K**L**VANNTRLW**V** | 65 | 0.6 | - |
| 100-109 | F**L**TS**E**LPQW**L** | 65 | 0.7 | - |
| 228-238 | F**L**ENFVRSSN**L** | 62 | 1.2 | - |
| 199-207 | K**L**VA**N**N**TRL** | 61 | 1.4 | + |
| 158-166 | G**M**GPSLIG**L** | 50 | 1.4 | + |
| 37-44 | Y**L**LDGLR**A** | 52 | 1.6 | - |
| 126-134 | S**M**AGSSAM**I** | 49 | 1.7 | - |
| 206-215 | R**L**WV**Y**CGNG**T** | 53 | 2 | - |
| 93-102 | Q**T**YK**W**E**T**FL**T** | 45 | 4 | - |
| 101-109 | L**T**S**E**LP**Q**W**L** | 53 | 6 | - |
| 5-13 | G**L**PVEYLQ**V** | 60 | 7 | - |
| 93-101 | Q**T**YK**W**E**TFL** | 51 | 7 | - |

| | | | | |
|---|---|---|---|---|
| ^{*} AA contributing to the HLA-A*0201 binding motif score are shown in bold. | | | | |
| ^{¶} Only peptides with HLA-*0201 binding affinities < 10 µM were tested for CTL recognition. | | | | |
| ^{§} Human HLA-A*0201-positive, H2-K^{b}-negative cells (JY) were used as target cells for CD8⁺ T cells from HLA-A2/K^{b} tg mice. | | | | |

### 2. Induction of M. tuberculosis-specific, HLA-A*0201-restricted CD8⁺ CTL in Ag85-DNA-immunized HLA-A2/K^{b} tg mice.

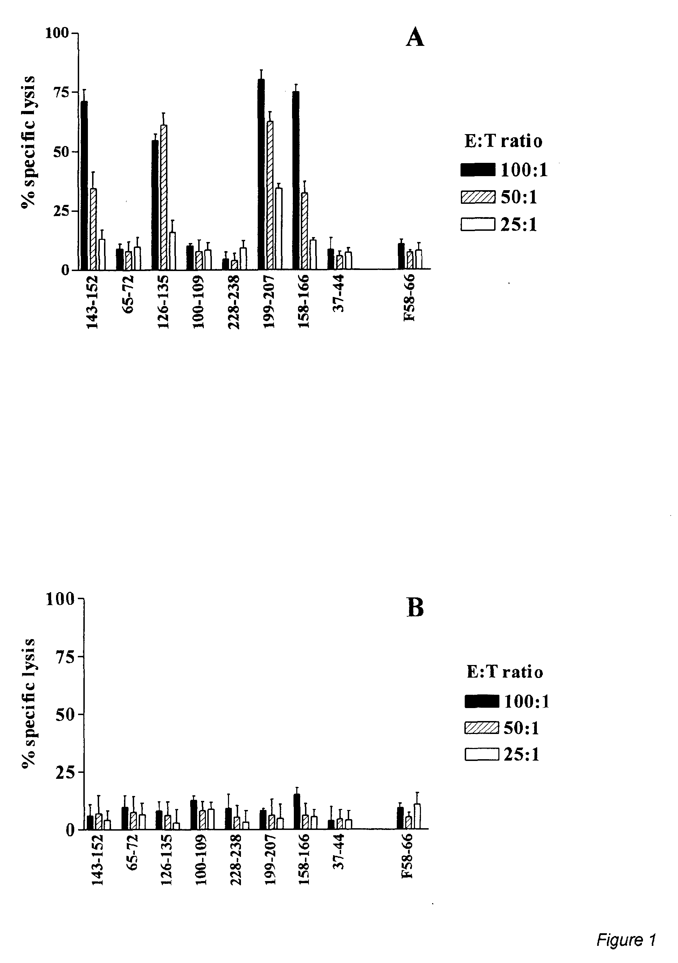

HLA-A*0201 transgenic mice represent a powerful model for the induction and examination of HLA-A*0201-restricted CD8⁺ CTL responses *in vivo* (14). To analyze CD8⁺ T cell responses against the major *M. tuberculosis* antigen, Ag85B, HLA-A2/K^{b} tg mice were immunized three times at three week intervals with Ag85B-encoding or control plasmid DNA. Two weeks after the last immunization, splenocytes were harvested, restimulated with a mix (5 µg/ml per peptide) of the 16 best binding Ag85B peptides (Table I), and analyzed one week later for their ability to lyse the human target cell JY, which expresses HLA-A*0201 but not H2-K^{b} or H2-D^{b}. Target cells were pulsed separately with each of the sixteen Ag85B peptides. Four of the 16 peptides (p143-152, p126-135, p199-207, and p158-166) were strongly recognized by CTL in a dose-dependent fashion (Fig. 1A). The fact that recognition was induced against only 4 out of 16 peptides, and the fact that many of the best binding peptides failed to be recognized by CD8⁺ T cells argues strongly against possible *in vitro* sensitization by the peptide mix used for restimulation. Similarly, no responses were found against the unrelated, HLA-A*0201-binding matrix protein epitope of influenza virus (p58-66) (Fig. 1A, 1B). As an additional control, to rule out such *in vitro* peptide sensitization, splenocytes from mice immunized with control vector DNA and stimulated with the same peptide mix, were tested as well. Cells from these animals failed to recognize any of the Ag85B peptides tested (Fig.1B).

The four thus defined HLA-A*0201-restricted Ag85B epitopes are strongly conserved between *M. tuberculosis* Ag85B and Ag85A, and the corresponding Ag85A peptides had comparable binding affinities for HLA-A*0201 (data not shown).

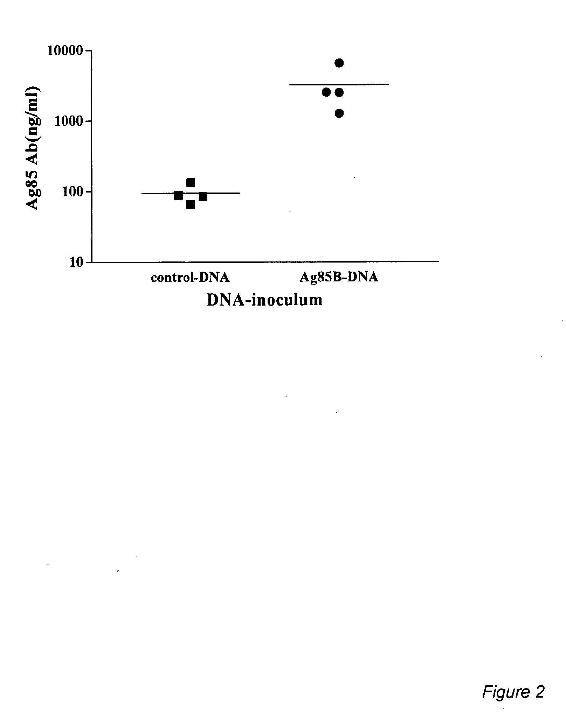

In addition to determining CTL activity, serum-antibodies against Ag85 were measured in Ag85-DNA immunized HLA-A2/K^{b} mice (Fig. 2). All four immunized mice produced significant levels of anti-Ag85 Ab, whereas control DNA immunized animals did not produce any anti-Ag85 Ab, demonstrating efficient induction of both cellular and humoral immunity in HLA-A2/K^{b} tg animals following Ag85 DNA immunization (Fig. 2).

### 3. Peptide immunization induces mycobacterium-specific, HLA-A*0201-restricted CD8⁺ T cells in HLA-A2/K^{b} tg mice.

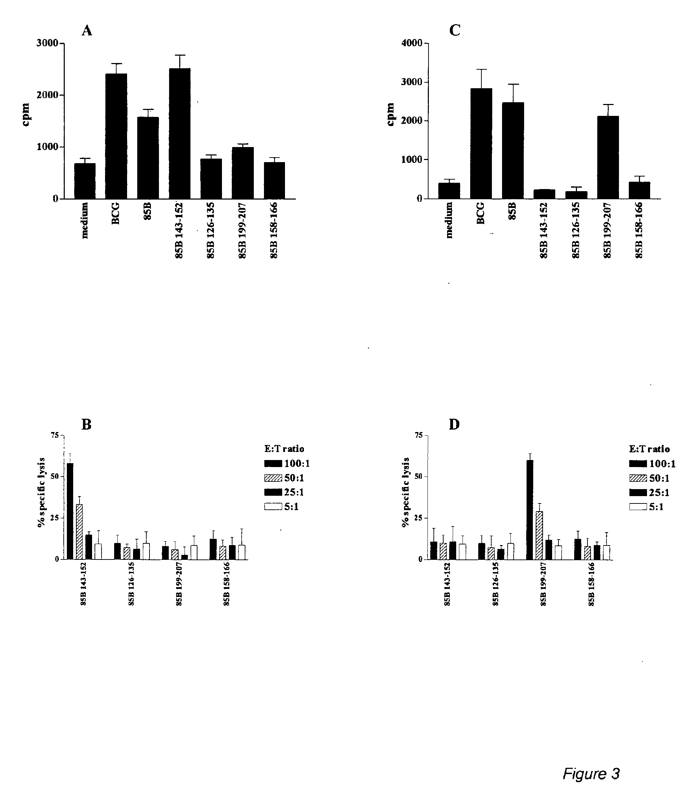

In order to determine whether the above defined Ag85B epitopes are naturally processed, and may represent candidate subunit components for anti-mycobacterial vaccination, HLA-A2/K^{b} mice were immunized separately with each of the four above identified Ag85B epitopes: p126-135, p143-152, p158-166 and p199-207. After 10 days splenocytes were depleted for CD4⁺ T cells and the remaining cells stimulated *in vitro* with various peptides, Ag85B protein or BCG (Fig. 3). CD8⁺ T cells from Ag85B p143-152- or p199-207-immunized mice proliferated (Fig. 3A,B) and produced IFN-γ (data not shown) in response to the immunizing peptide but not to control-peptide or any of the other Ag85B peptides. In addition, p143-152 or p199-207 immunized mice also responded strongly against the whole Ag85B protein as well as *M. bovis* BCG, indicating that natural processing of these CTL epitopes not only occurs following DNA immunization (Fig. 1), but also from BCG infected splenocytes. Unexpectedly, the two other Ag85B peptides identified by DNA immunization, p126-135 and p158-166, failed to induce any CD8⁺ T cell proliferation against Ag85B or BCG, while inducing proliferation against the immunizing peptides (data not shown). This indicates that DNA immunization could also induce T cells directed against peptides that are only processed following DNA plasmid immunization but not following whole antigen pulsing or BCG infection. Alternatively, some peptides may be tolerogenic rather than immunogenic, depending on the mode of delivery.

### 4. Identification of M. tuberculosis Ag85B-specific human CD8⁺ CTL in human HLA-A*0201⁺ individuals.

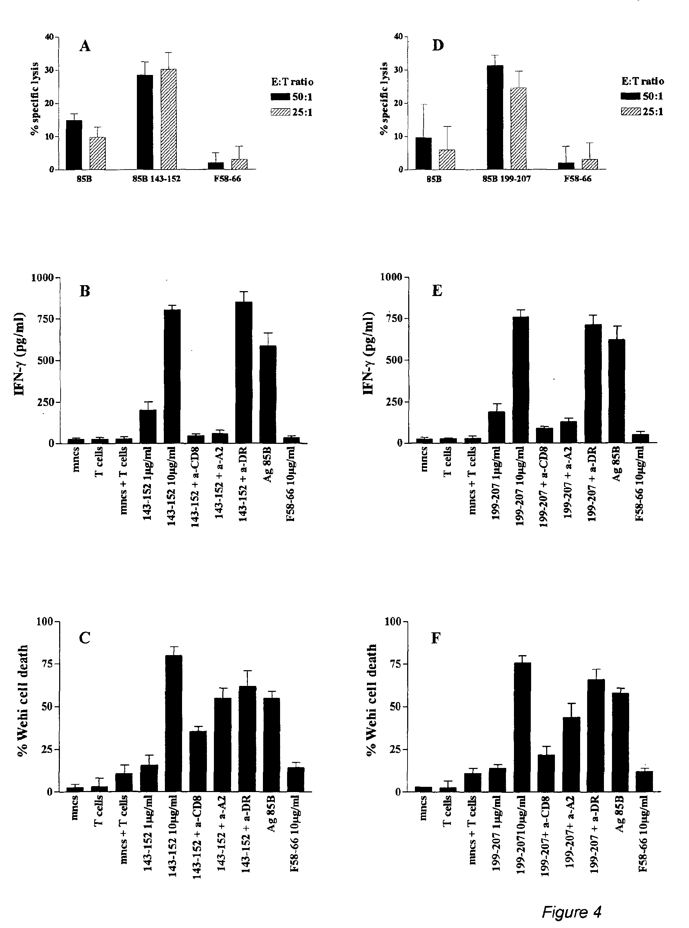

We next investigated whether CD8⁺ T cells, that recognize Ag85B p143-152 or p199-207 can be detected in the human repertoire in the context of HLA-A*0201. Stable CD8⁺ T cell lines were generated against either p143-152 or p199-207, using CD4-depletion and peptide pulsed autologous dendritic cells derived from HLA-A*0201⁺, BCG-responsive individuals. These T cell lines were able to lyse HLA-A*0201⁺ peptide-pulsed targets (Fig. 4A and 4D) and produced the pro-inflammatory cytokines IFN-γ (Fig. 4B and 4E) and TNF-α (Fig. 4C and 4F) in response to specific peptide only, whereas no responses were detected against other Ag85B peptides or the control HLA-A*0201 binding influenza A matrix peptide. Importantly, both CD8⁺ CTL lines were able to recognize whole Ag85B protein, demonstrating natural processing of both p132-152 and p199-207 by human cells (Fig. 4 A, B, C). Furthermore, T cell responses were CD8- and HLA-A*0201-dependent, since CD8⁺ T cell activation was inhibited by anti-CD8 mAb or anti-HLA-A*0201 mAb, but not anti-HLA-DR mAb.

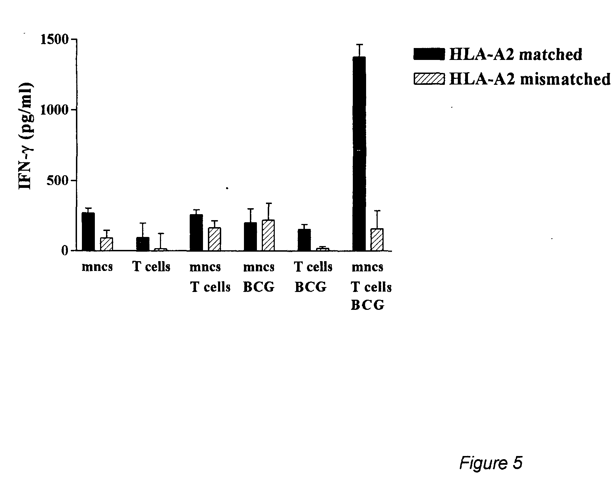

In order to determine whether such HLA class I restricted epitopes are also processed from mycobacterium infected macrophages, the major host cells harbouring mycobacterial pathogens, Ag85B p199-207-specific human CD8⁺ T cells were co-cultured with BCG-infected monocytes from HLA-A*0201⁺ or HLA-A*0201⁻ individuals. As shown in Figure 5, Ag85B p199-207-specific CD8⁺ T cells were able to produce IFN-γ upon co-culture with BCG-infected monocytes but not with uninfected monocytes or BCG alone. Moreover, cytokine production was only detected when the infected monocytes were matched for HLA-A*0201, indicating the HLA-dependency of the response (Fig. 5).

Taken together, these results show that CD8⁺, HLA-A*0201-restricted T cells directed against peptides of the immunodominant *M. tuberculosis* Ag85B are present in the human T cell repertoire, and effectively recognize mycobacterium-infected macrophages.

### 5. Detection of M. tuberculosis Ag85-specific, HLA-A*0201-restricted human T cells using Ag85B peptide/HLA-A*0201 tetramers.

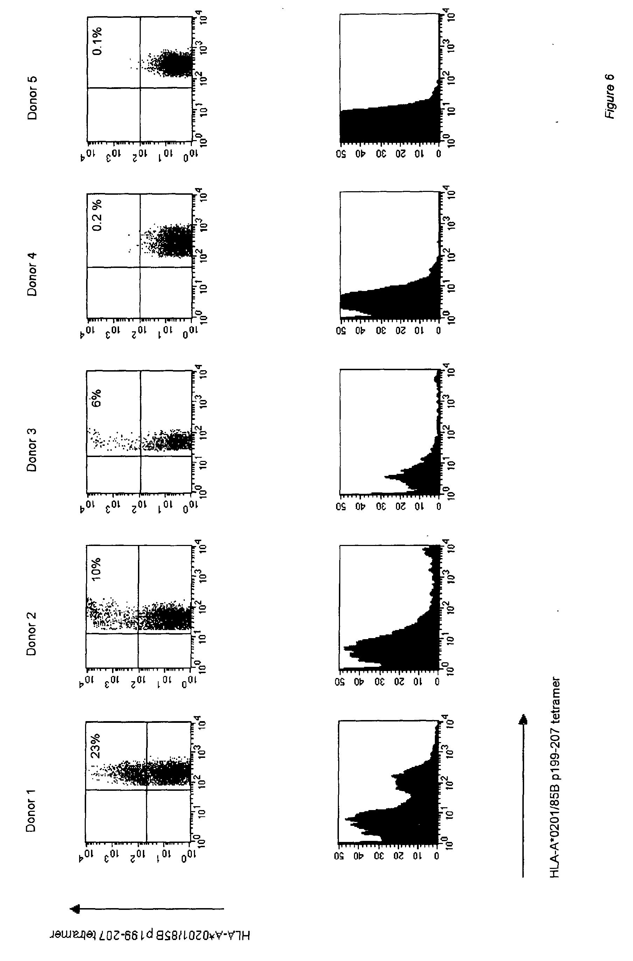

In order to visualize and enumerate human CD8⁺ T cells against *M. tuberculosis,* tetrameric complexes of HLA-A*0201 and Ag85B p199-207 were constructed using previously described procedures (23). Of the two Ag85B epitopes, p199-207 was chosen in view of the slightly more efficient recognition of this peptide compared to p143-152 by murine CD8⁺ T cells after DNA immunization (Fig.1A). As negative controls, HLA-A*0201 tetramers were used containing influenza A matrix peptide, HPV16 E7 peptide or no peptide at all. PE-labeled tetramers were used to identify and determine the frequency of p199-207- specific T cells in BCG-responsive donors. Virtually no specific staining could be detected in unstimulated, freshly isolated or frozen PBMC directly *ex vivo* probably due to the relative low precursor frequency of these cells among circulating PBMC in these healthy individuals (data not shown). However, in short term cultured, BCG-stimulated PBMCs (STCL) from these donors, CD8⁺ T cells were detected that bound to HLA-A*0201/Ag85B p199-207 tetramers, ranging from 6 - 23 % of the CD8⁺ T cell population (Fig. 6).

The observed tetramer staining was specific for HLA-A*0201 since STCL derived from HLA-A*0201⁻ PBMC did not show significant tetramer staining (Fig. 6, donor 4: 0.2%). Moreover, in HLA-A*0201⁺, BCG-non-responsive individuals no specific staining was observed (Fig. 6, donor 5: 0.1%). Furthermore, as additional controls, HLA-A*0201/Ag85B p199-207 tetramers did not stain HLA-A*0201-restricted T cells that recognize influenza A matrix p58-66 (data not shown), and BCG-induced STCLs did not bind to control tetramer complexes of HLA-A*0201 with HPV16 E7-derived control epitope or of HLA-A*0201 without any peptide (data not shown). Thus, the observed peptide/tetramer staining signals are specifically seen only in case of BCG responsiveness in the context of HLA-A2. These data thus showthat peptide/HLA tetramers are a novel tool for detection and enumeration of *M. tuberculosis*-specific CD8⁺ T cells.

### DESCRIPTION OF THE FIGURES

**Figure 1:** Cytotoxic activity of splenocytes derived from HLA-A2/K^{b} mice, immunized with Ag85B plasmid DNA (**A**) or with control-vector DNA (**B**). Two weeks after the last immunization, splenocytes were harvested, restimulated with a mix of the 16 best binding Ag85B peptides (final concentration 5 µg/ml per peptide), and analyzed one week later for their ability to lyse human JY target cells. Ag85B-derived peptides used to pulse HLA-A*0201⁺ target cells are indicated on the x-axis, in order of decreasing HLA-A*0201-binding affinity. The HLA-A*0201-binding peptide of influenza A matrix 58-66 (F58-66) (GILGFVFTL) was used as a control. E:T is effector to target ratio.

**Figure 2:** Quantification of serum antibodies to Ag85 in HLA-A2/K^{b} mice (4 mice/ group) following immunization with Ag85-encoding DNA or with control-vector DNA. The anti-Ag85 Ab titer is given on the *y*-axis (ng/ml).

**Figure 3:** Proliferative responses of (**A,C**) and specific lysis induced by **(B,D)** CD8⁺ splenocytes from Ag85B p143-152 **(A,B)** , or Ag85B p199-207 **(C,D)** immunized HLA-A2/K^{b} mice. Peptides used for *in vitro* challenge are indicated on the x-axis.

**Figure 4:** CTL activity **(A,D),** IFN-γ production **(B,E)** and TNF-α production **(C,F)** of human HLA-A*0201-restricted CD8⁺ T cells specific for Ag85B p143-152 **(A,B,C)** or Ag85B p199-207 **(D,E,F).** Peptides used for *in vitro* challenge are indicated on the x-axis. Influenza A matrix 58-66 (F58-66) was used as a negative control.

**Figure 5:** Human Ag85B 199-207-specific CTL efficiently recognize BCG-infected, HLA-matched (black bars), but not HLA-mismatched (hatched bars) monocytes. Ag85B p199-207-specific human CD8⁺ T cells were co-cultured with BCG-infected (MOI ≅ 2:1) monocytes from HLA-A*0201⁺ or HLA-A*0201⁻ individuals and IFN-γ levels were determined after 72 h in supernatants.

**Figure 6:** Specific staining of BCG-activated CD8⁺ T cells from BCG-responsive human donors using PE-labelled HLA-A*0201/Ag85B p199-207 tetramers. STCL from healthy, BCG-responsive, HLA-A*0201⁺ individuals (donor 1-3), from a HLA-A*0201⁻, BCG-responsive individual (donor 4) or from a HLA-A*0201⁺, BCG non-responsive individual (donor 5) were incubated with PerCP-labeled anti-CD8 mAb and PE-labeled tetrameric complexes of HLA-A*0201/Ag85B p199-207. PerCP-CD8 vs. PE-HLA-A*0201/Ag85B p199-207 plots are shown for the gated CD8⁺ population in the upper panel. Histograms of the events that fall in the boxed portions of the dot plots are shown in the lower panel. No specific staining was observed after incubation of BCG-induced STCLs with tetramers of HLA-A*0201/HPV16 peptide nor did HLA-A*0201/Ag85B p199-207 tetramers stain HLA-A*0201-restricted T cells that recognize influenza A matrix p58-66 (data not shown).

### REFERENCES

1. Dolin, P. J., M. C. Raviglione, and A. Kochi. 1994. Global tuberculosis incidence and mortality during 1990-2000. *Bull. W.H.O. 72: 213.*
2. Fine, P. E. M. 1995. Variation in protection by BCG: implications of and for heterologous immunity. *Lancet 346: 1339.*
3. Kaufmann, S. H. E. 1993. Immunity to intracellular bacteria. *Annu. Rev. Immunol. 11: 129.*
4. Geluk, A., V. Taneja, K.E. van Meijgaarden, E. Zanelli, C. Abou-Zeid, J.E.R. Thole, R.R.P. de Vries, C.S. David, and T.H.M. Ottenhoff. 1998. Identification of HLA class II-restricted determinants of *M. tuberculosis*-derived proteins using HLA-transgenic, class II deficient mice. *Proc. Natl. Acad. Sci. USA 95:10797.*
5. Ottenhoff, T. H. M., J. B. A. G. Haanen, A. Geluk, T. Mutis, B. Kale Ab, J. E. R. Thole, W. C. A. Van Schooten, P. J. Van den Elsen, and R. R. P. De Vries. 1991. Regulation of Mycobacterial heat-shock protein reactive T cells by HLA class II molecules: lessons from leprosy. *Immunol. Rev. 121:171.*
6. Flynn, J. L., M. M. Goldstein, K. J. Triebold, B. Koller, and B. R. Bloom. 1992. Major histocompatibility complex class I-restricted T cells are required for resistance to *Mycobacterium tuberculosis* infection. *Proc. Natl. Acad. Sci. USA 89: 12013.*
7. Stenger, S., R. J. Mazzaccaro, K. Uyemura, S. Cho, P. F. Barnes, J. P. Rosat, A. Sette, M. B. Brenner, S. A. Porcelli, B. R. Bloom, and R. L. Modlin. 1997. Differential effects of cytolytic T cell subsets on intracellular infection. *Science 276:1684.*
8. Tascon, R. E., M. J Colston,., S. Ragno, E. Stavropoulos, D. Gregory, and D. B. Lowrie. 1996. Vaccination against tuberculosis by DNA injection. *Nature Medicine 2: 888.*
9. Huygen, K., J. Content, O. Denis, D. L. Montgomery, A. M. Yawman, R. R. Deck, C. M. DeWitt, I. M. Orme, S. Baldwin, C. D'Souza, A. Drowart, E. Lozes, P. Vandenbussche, J.-P. Van Vooren, M. A. Liu, and J. B. Ulmer.1996. Immunogenicity and protective efficacy of a tuberculosis DNA vaccine. *Nature Medicine. 2:893.*
10. Kaleab, B., T. H. M. Ottenhoff, P. Converse, E. Halapi Genet Tadesse, M. Rottenberg, and R. Kiessling. 1990. Mycobacterial-induced cytotoxic cells as well as nonspecific killer cells derived from healthy individuals and leprosy patients. *Eur. J. Immunol. 20: 369.*
11. Turner, J., and H. M. Dockrell. 1996. Stimulation of peripheral blood mononuclear cells with live *Mycobacterium bovis* BCG activates cytolytic CD8⁺ T cells *in vitro. Immunology 87:339.*
12. Lalvani, A., R. Brookes, R. J. Wilkinson, A. S. Malin, A. A. Pathan, P. Andersen, H. Dockrell, G. Pasvol, and A. V. S. Hill. 1998. Human cytolytic and interferon γ-secreting CD8⁺ T lymphocytes specific for *Mycobacterium tuberculosis. Proc. Natl. Acad. Sci. USA 95: 270.*
13. Mohagheghpour, N., D. Gammon, L.M. Kawamura, A. van Vollenhoven, C.J. Benike, and G. Engleman. 1998. CTL response to Mycobacterium tuberculosis: identification of an immunogenic epitope in the 19-kDa lipoprotein. *J. Immunol 161:2400.*
14. Vitiello, A., D. Marchesini, J. Furze, L. A. Sherman, and R. W. Chesnut. 1991. Analysis of the HLA-restricted influenza-specific cytotoxic T lymphocyte response in transgenic mice carrying a chimeric human-mouse class I major histocompatibility complex. *J. Exp. Med 173: 1007.*
15. D'Amaro, J., J. G. A. Houbiers, J. W. Drijfhout, R. M. P. Brandt, R. Schipper, J. N. Bouwes Bavinck, C. J. M. Melief and W. M. Kast. 1995. A computer program for predicting possible cytotoxic T lymphocyte epitopes based on HLA class I peptide binding motifs. *Hum. Immunol. 43:13.*
16. Tan, T. L. R., A. Geluk, M. Toebes, T. H. M. Ottenhoff, and J. W. Drijfhout. 1997. A novel, highly efficient peptide-HLA class I binding assay using unfolded heavy chain molecules: identification of HIV-1 derived peptides that bind to HLA-A*0201 and HLA-A*0301. *J. Immunol. Methods 205:201.*
17. Studier, F. W., and B. A. Moffatt. 1986. Use of bacteriophage T7 RNA polymerase to direct selective high-level expression of cloned genes. *J. Mol. Biol. 189:113.*
18. Franken, K. L. M. C., H. S. Hiemstra, K. E. van Meijgaarden, Y. Subronto, J. den Hartigh, T. H. M. Ottenhoff, and J. Drijfhout. 2000. Purification of His-tagged proteins by immobilized chelate affinity chromatography (IMAC): the benefits from the use of organic solvent. *Protein expression and Purification. 18:95.*
19. De Wit, L., M. Palou, and J. Content. 1994. Nucleotide sequence of the 85B-protein gene of *Mycobacterium bovis* BCG and *Mycobacterium tuberculosis. DNA Sequence 4: 267-270.*
20. Okada, C.Y., and M. Rechsteiner. 1982. Introduction of macromolecules into cultured mammalian cells by osmotic lysis of pinocytic vesicles. *Cell 29: 33.*
21. Romani, N., D. Reider, M. Heuer, S. Ebner, E. Kampgen, B. Eibl, D. Niederwieser, and G. Schuler. 1996. Generation of mature dendritic cells from human blood. An improved method with special regard to clinical applicability. *J. Immunol. Methods 196:137.*
22. Traversari, C., P. van der Bruggen, B. Van den Eynde, P. Hainaut, C. Lemoine, N. Ohta, L. Old, and T. Boon. 1992. Transfection and expression of a gene coding for a human melanoma antigen recognized by autologous cytolytic T lymphocytes. *Immunogenetics 35:145.*
23. Altman, J. D., P. A. H. Moss, P. J. R. Goulder, D. H. Barouch, M. G. McHeyzer-Williams, J. I. Bell, A. J. McMichael, and M. M. Davis. 1996. Phenotypic analysis of antigen-specific T lymphocytes. *Science 274:94.*
24. Sette, A., A. Vitiello, B. Reherman, P. Fowler, R. Nayersina, W. M. Kast, C. J. Melief, C. Oseroff, L. Yuan, J. Ruppert, and H. M. Grey. 1994. The relationship between class I binding affinity and immunogenicity of potential cytotoxic T cell epitopes. *J. Immunol. 153:5586.*

## Claims

1. A peptide derived from an Ag85 protein of mycobacterium comprising 8-11 amino acid residues, said peptide capable of inducing proliferation of MHC class I-restricted CD8⁺ T cells *in vivo.*

2. A peptide comprising a peptide according to claim 1 flanked by amino acids representing antigen processing sites.

3. A polypeptide comprising at least 2 peptides according to claim 2.

4. A peptide or polypeptide according to anyone of claims 1 to 3 wherein said peptide is derived from an Ag85B protein.

5. A peptide or polypeptide according to anyone of claims 1 to 4 wherein said Ag85 protein of mycobacterium is selected from the group consisting of M. tuberculosis, M. leprae, M. bovis, M. ulcerans and M. avium.

6. A peptide or polypeptide according to anyone of claims 1 to 5 wherein said MHC class I-restricted CD8⁺ T cells are HLA-A*0201- restricted CD8⁺ T cells.

7. A nucleic acid encoding a peptide or a polypeptide according to anyone of claims 1 to 6.

8. A vector comprising a nucleic acid according to claim 7.

9. A host cell comprising a nucleic acid according to claim 7 or a vector according to claim 8.

10. Use of a peptide or polypeptide according to anyone of claims 1 to 6, a nucleic acid according to claim 7, a vector according to claim 8 for the preparation of a vaccine against mycobacterium.

11. A method to detect and/or enumerate CD8⁺ T cells against mycobacterium comprising tetrameric complexes of MHC class I and a peptide or polypeptide according to anyone of claims 1 to 6.
